# EUROPEAN PATENT SPECIFICATION

(11) **EP 3 352 108 B1**
(45) Date of publication and mention of the grant of the patent: **06.10.2021**
(21) Application number: 18154034.5
(22) Date of filing: 11.08.2010
(51) Int. Cl.: G16H 40/63

(54) **GRAPHICAL INTERFACE FOR ANALYTE METER**
GRAFISCHE SCHNITTSTELLE FÜR EINEN ANALYTMESSER
INTERFACE GRAPHIQUE POUR INSTRUMENT DE MESURE D'ANALYTE

(30) Priority: 11.08.2009 US 233113 P
(43) Date of publication of application: 25.07.2018
(62) Divisional of application: 10752465.4
(73) Proprietor: Ascensia Diabetes Care Holdings AG, 4052 Basel (CH)
(72) Inventor: GAONKAR, Kripa, Dobbs Ferry, NY 10522 (US); RIPLEY, Paul M., Nanuet, NY 10954 (US); SUN, Hoi-Cheong, Steve, Mount Kisco, NY 10549 (US); TELSON, Stan, Roseville, CA 95747 (US); WU, Mu, Hopewell Junction, NY 12533 (US); DUNNE, Nancy, Sleepy Hollow, NY 10591 (US)
(74) Representative: Cohausz & Florack

(56) References cited:
- EP-A2- 1 457 913
- US-A1- 2003 176 183

## Description

### FIELD OF THE INVENTION

The present invention relates generally to methods and systems for presenting information to a user of a diagnostic system. More specifically, the methods and systems according to aspects of the present invention provide a graphical user interface for a diagnostic system. Additionally, the graphical user interface provides information for operating the diagnostic system.

### BACKGROUND OF THE INVENTION

The quantitative determination of analytes in body fluids is of great importance in the diagnoses and maintenance of certain physiological abnormalities. For example, lactate, cholesterol and bilirubin are monitored in certain individuals. In particular, it is important that individuals with diabetes frequently check the glucose level in their body fluids to regulate the glucose intake in their diets. The results of such tests can be used to determine what, if any, insulin or other medication needs to be administered.

Diagnostic systems, such as blood-glucose systems, may employ a meter or instrument to calculate the concentration of an analyte in a sample of body fluid. In some types of diagnostic systems, test sensors are used to test a sample of blood. A test sensor contains biosensing or reagent material that reacts with the analyte, e.g., blood glucose, in the sample. For example, the testing end of the sensor may be placed into contact with the fluid being tested (e.g., blood) that has accumulated on a person's finger after the finger has been pricked. A sufficient amount of fluid to be tested may be drawn from the testing end by capillary action to the reagent material in the sensor. The meter receives the test sensor and applies optical or electrochemical testing methods to by measure an output, such as current or color, from the reaction between the analyte and the reagent in the test sensor. Diagnostic systems typically employ a graphical user interface to display the results of the testing to the user. The graphical user interface may also be employed to display instructions to the user.

Diagnostic systems require the user to complete several steps during the testing procedure. The accuracy of such testing methods, however, depend on the manner in which the user completes the steps. Document EP 1 457 913 A2 relates to a method of managing data for a plurality of analyte test instruments connected to a data communication network comprising the steps of: detecting via host computer the connection of each instrument to the data communication network; uploading data received from each instrument to the host computer; processing the upload data on the host computer for operational review; and downloading configuration data from the host computer to each test instrument, the downloaded data comprising instrument specific set up and control data.

### SUMMARY OF THE INVENTION

In view of the foregoing, it would be desirable to have systems and methods that provide a diagnostic system with a graphical user interface that provides users with clear and easy-to-follow instructions for conducting the testing procedure of diagnostic systems to produce accurate results. Moreover, it would be desirable to have a graphical user interface that provides users with instructions on how to operate the diagnostic systems when an error or exceptional condition arises. The objects are solved according to the present invention by a blood-glucose meter according to claim 1.

Accordingly, diagnostic systems according to aspects of the present invention include a meter that is configured to receive a test sensor during a testing procedure. The diagnostic systems also include a computing device coupled to the meter. The test sensor receives a fluid sample during the testing procedure. The meter includes a measurement system that determines a measurement of a concentration of an analyte in the fluid sample. The computing device receives and processes the measurement from the meter. In particular, the computing device has enhanced processing and presentation capabilities that provide visual and/or audio instructions on how to operate the diagnostic systems, especially when an error or exceptional condition arises.

Diagnostic systems according to aspects of the present invention employ a graphical user interface (GUI), or display, that provides clear and easy-to-follow instructions for conducting the testing procedure. For example, a processing device in a diagnostic system executes software that is stored on computer-readable media to present illustrative graphics, textual information, and/or audio on a corresponding user interface for each step during the testing procedure. As such, the user receives clear step-by-step instructions to minimize the chance of user error during the testing procedure. In some embodiments, the software may enhance the presentation of instructions by employing animation.

In some embodiments, the GUI also presents illustrative graphics, textual information, and/or audio that guide the user through appropriate steps when an error or exceptional condition occurs during the testing procedure. For example, because the result of the chemical reaction between the analyte and a reagent on the test sensor may vary at different temperatures, the accuracy of the testing procedure may be affected by the temperature of the test sensor. Although the actual measurement may be corrected based on the actual test sensor temperature taken right before the reaction begins, in some cases, the accuracy of the testing procedure is improved by replacing the test sensor with one that has a temperature within a preferred range. Thus, in some embodiments, the GUI presents illustrative graphics, textual information, and/or audio that instruct the user to replace the test sensor when the diagnostic system senses that the test sensor temperature is outside a preferred range.

Although the meter may include the processing device, the software, and the GUI for presenting the illustrative graphics, textual information, and/or audio, it is understood that diagnostic systems according to the aspects of the present invention employ a variety of architectures. For example, a diagnostic system employs a meter in combination with an external device, such as a conventional personal computer, a personal data assistant (PDA), or smart phone. As such, the software is loaded on the external device to allow a processor of the external device to execute the software and to present illustrative graphics, textual information, and/or audio on a user interface of the external device. Indeed, in some embodiments, the software is a part of an data management system that is executed on the external device to manage, analyze, and present test results that have been stored by the meter. Is such embodiments, the data management system takes advantage of greater processing and display capabilities to provide enhanced functionality, which may not be otherwise possible with the processor and user interface on a meter.

### Brief Description of the Drawings

FIG. 1 illustrates an embodiment of a diagnostic system including meter and a computing device according to aspects of the present invention.
FIG. 2A illustrates a connection between a meter and a computing device.
FIG. 2B illustrates a connection between a meter and a computing device that provides secondary isolation/protection from the power source of the computing device, according to aspects of the present invention.
FIG. 2C illustrates another connection between a meter and a computing device that provides secondary isolation/protection from the power source of the computing device.
FIG. 3 illustrates a miniature blood glucose meter according to aspects of the present invention.
FIG. 4 illustrates the miniature blood glucose meter coupled to a smart phone according to aspects of the present invention.
FIGS. 5A-E illustrates example graphical and textual information that may be displayed by a diagnostic system according to aspects of the present invention.
FIG. 6 illustrates an alternative embodiment of a meter according to aspects of the present invention.
FIG. 7 illustrates another alternative embodiment of a meter according to aspects of the present invention.
FIG. 8 illustrates yet another alternative embodiment of a meter according to aspects of the present invention.

### DESCRIPTION OF ILLUSTRATED EMBODIMENTS

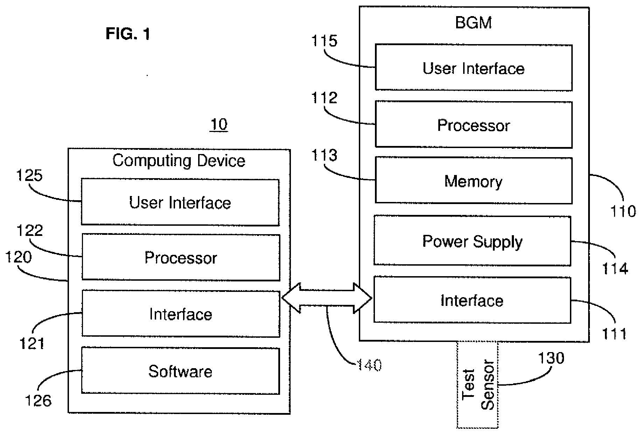

FIG. 1 provides a non-limiting example of a diabetes-management system 10, which allows individuals to actively monitor and record measurements of their blood glucose concentration. As shown in FIG. 1, the diabetes-management system 100 includes a blood-glucose meter (BGM) 110 and a computing device 120. A connection 140 allows the meter 110 to communicate with the computing device 120. The meter 110 obtains point-in-time measurements of blood-glucose concentrations in blood samples and communicates the measurement data to the computing device 120. The computing device 120 executes data management software 126 (executable program instructions stored on computer-readable media) to process the measurement data from the meter 110.

As illustrated in FIG. 1, the meter 110 engages a test sensor 130, which receives a blood sample for analysis. For example, a user may employ a lancing device to pierce a finger or other area of the body to produce a blood sample at the skin surface. The user may then collect the blood sample by placing the test sensor 130 into contact with the sample. As is well known in the art, the test sensor may be an electrochemical test sensor or an optical test sensor.

The meter 110 includes a reaction-detection system for measuring the glucose concentration of the blood sample collected by the test sensor 130. For example, the reaction-detection system may include contacts for the electrodes to detect the electrochemical reaction for an electrochemical test sensor. Alternatively, the reaction-detection system may include an optical detector to detect the chromatic reaction for an optical test sensor. To calculate the actual concentration of analyte from the electrochemical or chromatic reaction measured by the reaction-detection system and to generally control the procedure for testing the sample, the meter 110 employs at least one processor 112, which executes programmed instructions according to a measurement algorithm. Data processed by the processor 112 is stored in a memory 113. Furthermore, the meter 110 includes a user interface 115 that includes a display that shows information regarding the test results.

The computing device 120 may be selected from a variety of processing devices, such as desktop or laptop personal computers (PCs), handheld or pocket personal computers (HPCs), compatible personal digital assistants (PDAs), and smart phones. The processing devices may employ a variety of operating systems and configurations. For example, if the computing device 120 is a desktop or laptop personal computer, the operating system may be a version of Microsoft® Windows® or Apple® Mac® OS. Alternatively, if the computing device 120 is a smart phone, the operating system may correspond with Blackberry® devices from Research in Motion Limited or iPhone® from Apple®.

The computing device 120 includes a processor 122 that is capable of receiving and executing any number of programmed instructions provided on computer-readable media. In addition, the computing device 120 includes a user interface 125 for displaying graphics, text, and/or other audiovisual content. The user interface 125 may be incorporated into the housing of the computing device 120, but it is understood that the user interface 125 may be a separate component, such as a display monitor, that is coupled to the computing device 120.

Although the meter 110 stores test results and provides a user interface 115 to display test results, the test results collected by the meter 110 are communicated to the computing device 120 for additional processing by the data management software 126 and display by the user interface 125. The software 126 on the computing device 120 provides more advanced functionality for managing, processing, and displaying test results and related information. In addition, the computing device 120 provides an enhanced user interface 125 that provides advanced visual and/or audio presentation capabilities. In addition to providing high resolution graphics, the computing device 120 may also allow information to be communicated to the user via audio signals. Moreover, the computing device 120, through network connectivity, may provide the diagnostic system 10 with access to other functionality and data sources.

In general, the computing device 120 may provide processing and presentation capabilities that are not available with the meter 110. It is noted, however, that the meter 110 can fully operate to measure and display an analyte concentration when it is not connected to the computing device 120.

As shown in FIG. 1, the meter 110 includes a communications interface element 111 that enables the meter 110 to connect with the communications interface element 121 of the computing device 120. The communications interface elements 111 and 121 employ wired or wireless interface technologies, such as USB or Bluetooth® technology, to make the devices compatible and enable the appropriate data connections.

As shown further in FIG. 1, the meter 110 includes a power supply 114. The power supply 114 may be a lithium-ion rechargeable battery that receives recharging power from the computing device 120 via the connection 140. In this embodiment, the connection 140 between the meter 110 and the computing device 120 includes signal line connections as well as DC power line connections that allow the meter 110 to draw low voltage current from the computing device 120.

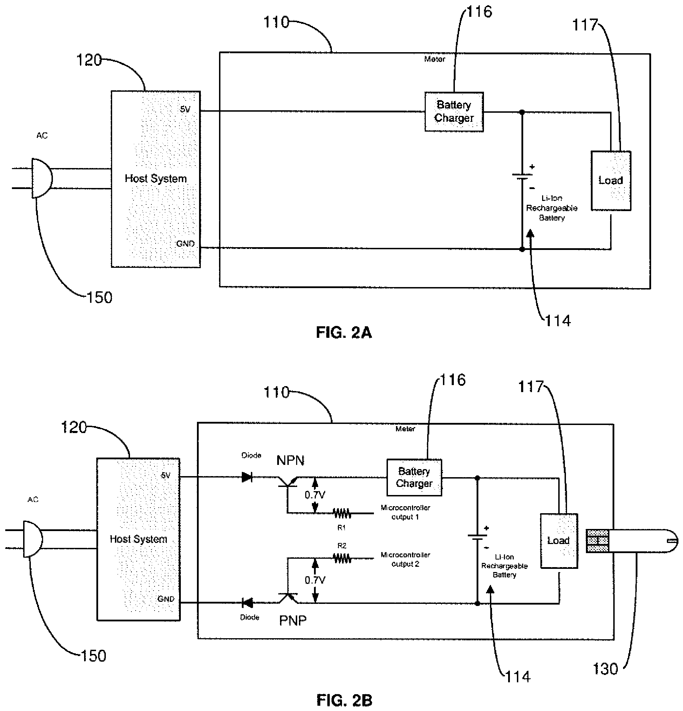

As such, some embodiments employing power line connections between the meter 110 and the computer device 120 protect the user against the danger of electric shock from the power source of the computing device 120. These embodiments provide such protection particularly when the user conducts a test while the meter 110 remains physically connected to the computing device 120. As shown in FIG. 2A, the computing device 120 can draw power from an AC source 150. The computing device 120 is normally isolated from the AC power lines to protect the user. This feature is called primary isolation and protection. However, if the primary isolation/protection for the computing device 120 fails, electricity from the AC power lines can be unsafely delivered via the computing device 120 to the user. Thus, when the meter 110 is plugged into the computing device 120 and the user performs a test with the test strip 130, the user may be shocked when touching the tip of the test strip 130. As a secondary precaution, testing can be disabled when the meter 110 is connected to the computing device 120. However, it is inconvenient for the user to disconnect the meter 110 from the computing device 120 each time to perform a test and to reconnect the meter 110 to transfer test results to the computing device 120. To eliminate this inconvenience, aspects of the present invention electrically isolate the meter 110 from the computing device 120 but allow the meter 110 to remain mechanically connected to the computing device 120 during testing.

In some embodiments, the meter 110 is electrically isolated from power of the computing device 120 through switches and other standard isolation techniques, such as an isolated DC-DC converter. Isolated DC-DC converters normally use a small transformer, called a flyback transformer. Advantageously, isolated DC-DC converters allow the user to conduct testing with the meter 110 while it remains mechanically connected to the computing device 120.

FIGS. 2B-C illustrate example connections between the meter 110 and the computing device 120 that electrically isolate the meter 110 from the power of the computing device 120, particularly when the primary isolation/protection for the computing device fails.

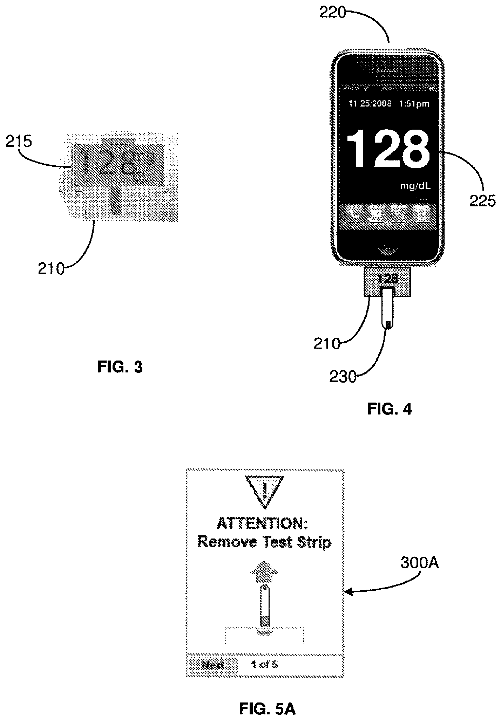

Referring to FIG. 2B, two transistors NPN and PNP during normal operation are turned on when particular voltages are supplied by the microcontroller on the meter 110 to the base input of the transistors NPN and PNP. For example, when the voltage between the base and emitter of the NPN transistor is over 0.7 V, the NPN transistor is turned on. When the voltage between the base and emitter is over -0.7 V, the PNP transistor is turned on. The corresponding two diodes shown in FIG. 4 are forwardly biased so that the current can flow through the diodes when the transistors are turned on. The rechargeable battery 114 inside the meter 110 can then be charged via the battery charger 116. The microcontroller and other electronics inside the meter 110 are shown collectively as load 117 in FIG. 4 because they draw power from the battery 114.

Referring still to FIG. 2B, when the test strip 130 is received by the meter 110, the microcontroller turns off the transistors, and the meter 110 operates solely on power from the battery 114. When the primary isolation/protection breaks down, the two power lines (labeled as 5V and GND) can carry the power line voltages. Therefore, the voltage relative to the meter 110 can be as high as 310 peak volts. The voltage can be positive and negative. When the high voltage is negative, the diodes are inversely biased and do not conduct current. When the high voltage is positive, the NPN and PNP transistors are turned off and do not conduct current. (Diodes or transistors alone will not provide sufficient protection when the polarity of the high voltage changes, as in the case of an AC power lines.) The diodes and the transistors need to have a break down voltage of over 400 V to achieve adequate protection for the user. The values for resistors R1 and R2 shown in FIG. 2B should be sufficiently large. Their values are determined together with the hFE (amplification) of the transistors to provide sufficient protection to the user during normal operation and in the event of a break down of the primary isolation/protection.

Compared to other isolation/protection techniques, such as an isolated DC-DC converter, the embodiment of FIG. 2B can advantageously be implemented at a lower cost and with a smaller geometry, i.e., requiring less board space on the meter 110. The secondary isolation/protection does not necessarily have to be designed with the same level of protection as the primary isolation/protection thus minimizing cost is possible.

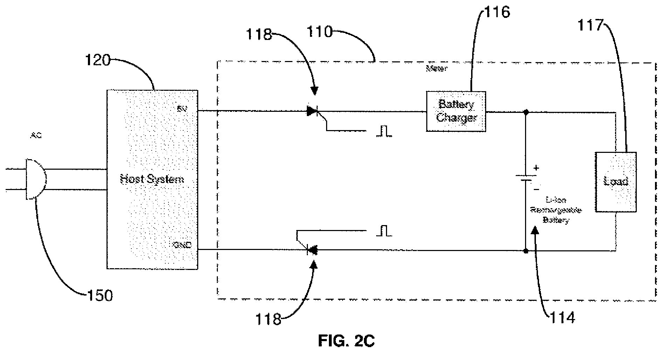

Referring to FIG. 2C, thyristors 118, such as silicon controlled rectifiers (SCRs), are employed for secondary isolation/protection. When the proper activation signal (short pulse) is applied to the gate input, the SCR 118 turns on. There is no need to turn the SCR 118 off, because when there is no AC line failure, the charge current maintains the on-state of the SCR 118. In the event of AC line failure, the AC changes polarity every 1/50 (Europe) or 1/60 seconds (North America) and the SCR turns off when the polarity is reversed thereby protecting the user.

In sum, aspects of the present invention automatically electrically isolate the meter 110 from the power source of a coupled computing device 120 when the user begins to conduct a test with the meter 110, e.g., when the user inserts the test strip 130 into the meter 110. This feature protects the user from failure of the primary isolation/protection for the computing device 120.

As described previously, the computing device 120 may be selected from a variety of processing devices, including portable computing devices. FIG. 3 illustrates a highly portable miniature meter 210 that is compatible with portable computing devices. The size of the miniature meter 210 allows it to be easily coupled to a portable computing device, such as a PALM® handheld, a Blackberry® device, or an Apple® iPhone® device, via a physical connection. For example, the miniature meter 210 may be approximately 20 mm × 15 mm × 5 mm in size. The miniature meter 210 includes a user interface 215, which, for example, may employ graphic liquid crystal display (LCD) or organic light-emitting diode (OLED), segment LCD or OLED, or the like. The graphical user interface 215 may have an area up to approximately 40% of a face of the miniature meter 210 and may have a thickness of approximately 0.3 mm.

As shown in FIG. 4, the miniature meter 210 measures the analyte concentration of the sample on a test sensor. The miniature meter 210 stores the analyte concentration and the analyte concentration can be displayed on the user interface 215. For example, the miniature meter 210 stores a minimum of one week of test results. This memory requirement is lower than other meters. For instance, at four tests per day, there are 28 test results for a week. Each test result requires approximately 8 bytes of storage so the total memory required would be approximately 224 bytes.

As further illustrated in FIG. 4, the miniature meter 210 is coupled to an Apple® iPhone® device 220. The Apple® iPhone® device 220 provides more advanced functionality for managing, processing, and displaying test results and related information. In particular, the Apple® iPhone® device 220 includes a user interface 225 that displays information based on the data received from the miniature meter 210.

Generally, the computing device 120 executes data management software 126 and presents data and information relating to the meter 110 on the user interface 125 of the computing device 110. Moreover, the computing device 120 can present the data and information while the meter 110 remains coupled to the computing device during testing. In particular, the user interface 125 presents clear and easy-to-follow instructions for conducting the testing procedure on the meter 110. For example, the computing device 120 executes software 126 that is stored on computer-readable media to present illustrative graphics, textual information, and/or audio for each step during the testing procedure. As such, the user receives clear step-by-step instructions to minimize the chance of user error during the testing procedure. In some embodiments, the software 126 may enhance the presentation of instructions by employing animation. For example, the user interface 125 may show an animated person or character (e.g., a cartoon depiction of a health care provider, diabetes care educator, the user, or a person of the user's choice) to guide the user through the steps in a more engaging and personable manner.

In some embodiments, the user interface 125 also shows illustrative graphics, textual information, and/or audio that guide or assist the user through appropriate steps when an error or exceptional condition occurs during the testing procedure. For example, the temperature of the reagent on the test sensor 130 may affect the accuracy of the concentration of analyte calculated by the meter, as the level of reaction between the analyte and the reagent may be dependent on the temperature of the reagent. As such, some embodiments of the present invention determine a temperature for the reagent and use this calculated temperature to produce a more accurate measurement of the analyte concentration. In particular, the meter 110 has a temperature-measuring system which provides a calculated temperature as a variable input for a measurement algorithm. Although the actual measurement is corrected based on the actual test sensor temperature, in some cases, however, the accuracy of the testing procedure is improved by replacing the test sensor 130 with one that has a temperature within a preferred range, e.g., closer to the ambient temperature. Thus, when the temperature-measuring system determines that the temperature of the test sensor 130 is not in an acceptable range, the user interface may present illustrative graphics and textual information (as well as audio) that instruct the user to replace the test sensor when the diagnostic system senses that the test sensor temperature is outside a preferred range. Examples of such illustrative graphics and textual information are shown in FIGS. 5A-E.

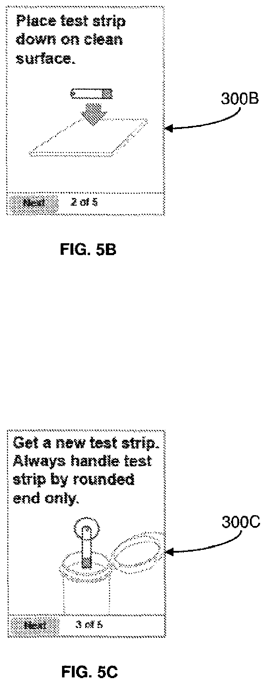

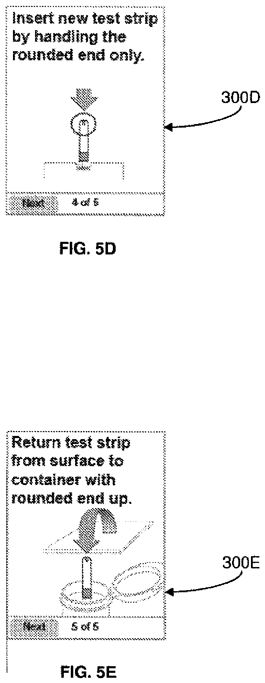

The illustrative graphics and textual information on the screen 300A shown in FIG. 5A alerts the user to an exceptional condition regarding the test sensor 130 and instructs the user to remove the test sensor 130. The screen 300B in FIG. 5B then instructs the user to place the removed test sensor 130 on a clean surface. As shown in FIG. 5C, the screen 300C instructs the user to retrieve another test sensor from a test sensor container, reminding the user how to handle the test sensors 130. Because the removed test sensor 130 has been placed on the clean surface, the user cannot accidentally select the removed test sensor 130 from the container. The screen 300D in FIG. 5D then instructs the user to insert the new test sensor into the meter 110, reminding the user again how to handle the test sensors 130. As shown in FIG. 5E, the screen 300E finally instructs the user to return the removed test sensor 130 to the test sensor container for later use.

The user may step through the sequence of screens 300A-E corresponding to FIGS. 5A-E by operating the "Next" pushbutton when the user is ready to move to the subsequent screen. Alternatively, the user interface 125 may show an automated slideshow that loops through screens 300A-E. Alternatively, as discussed previously, the graphical information in screens 300A-E may be shown as an animated presentation.

While it may be advantageous to present the illustrative graphics, textual information, and/or audio during the actual testing procedure or operation of the meter 110, it is understood that the illustrative graphics, textual information, and/or audio may be shown separately as a tutorial. Furthermore, it is understood that the user interface 125 may provide any information that may guide or assist the user in the operation of the meter and is not limited to presenting the types of information shown in screens 300A-E. For example, the user interface 125 may present screens that guide a user through a migration from one type of meter to another; such a feature would promote loyalty to a particular brand or line of meters.

As described previously, the computing device 120 includes data management software 126. The software 126 on the computing device 120 includes a collection of programs or computer code that receives and processes data measured by the meter 110. The software 126 processes and/or displays this input in a manner that is desired by the user. This information may be used by, for example, a user, home care provider (HCP), and/or a physician. Advantageously, the software 126 can provide the advanced displays and data processing that may be required by a user who tests multiple times a day (e.g., about six to about ten times a day). For example, the software 126 may include a product similar to WINGLUCOFACTS® Diabetes Management Software available from Bayer HealthCare LLC (Tarrytown, New York). As such, the software 126 may provide a complete tool kit that receives and stores test results from a blood-glucose measurement system, receives and stores other testing information such as test times and meal markers, tracks test results in an electronic logbook, calculates averages and provides statistical analysis of outlier test results, summarizes and provides feedback on the test results, provides a customizable graphical user interface (GUI), displays user-friendly charts and graphs of the test results, tracks test results against user-specific target ranges, provides predictive analysis, and/or sends data to healthcare professionals via fax, email, etc.

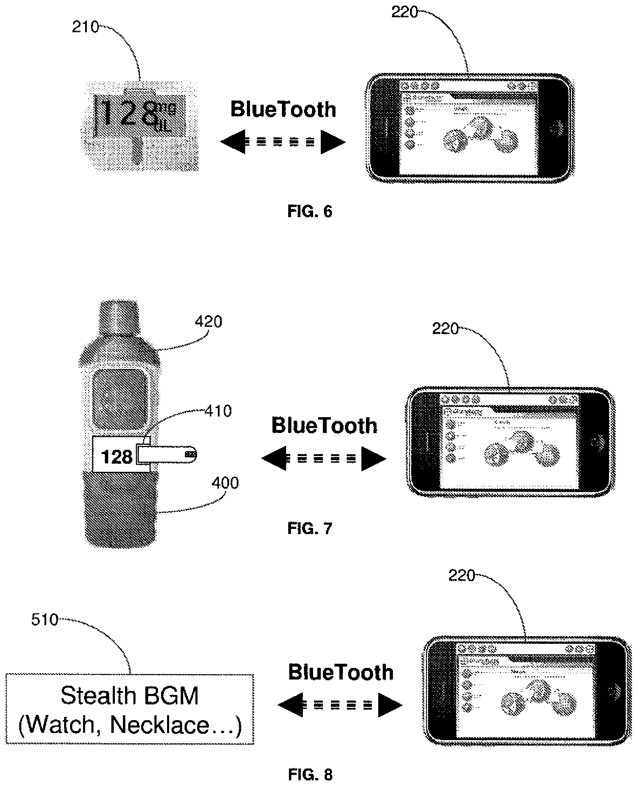

Diagnostic systems according to the aspects of the present invention employ a variety of architectures and configurations. As described previously with reference to FIG. 4, a meter is configured as a miniature meter 210 that is highly portable and that can be coupled to a portable computing device, such as the Apple® iPhone® 220. In an alternative embodiment, however FIG. 6 illustrates the miniature meter 210 communicating wirelessly, e.g., via Bluetooth®, with the Apple® iPhone® 220. As shown in FIG. 5, the Apple® iPhone® 220 is executing data management software.

In another alternative embodiment, FIG. 7 illustrates a meter that is configured as a miniature meter component 410 of an integrated lancet device 400. The integrated lancet device 400 combines a lancet 420 for producing a sample at a skin surface with a meter 410 for analyzing the sample. The meter 410 may be integral with the lancet 420 or may be removably coupled to the lancet 420. By way of example, the meter 410 in FIG. 7 communicates wirelessly, e.g., via Bluetooth®, with the Apple® iPhone® 220. However, the communication may be via a wired connection.

In yet another embodiment, FIG. 8 illustrates a meter that is configured as a "stealth" meter 510 that is discretely configured as a watch, necklace, or the like. By way of example, the "stealth" meter 510 in FIG. 8 communicates wirelessly, e.g., via Bluetooth®, with the Apple® iPhone® 220. However, the communication may be via a wired connection.

It is understood that the meter 110, rather than the computing device 120, may be employed to execute its own software and present information, such as that shown in screens 300A-E of FIGS. 5A-E. This is particularly advantageous in embodiments that do not allow any communication between the meter 110 and the computing device 120 when testing is being conducted with the meter.

Furthermore, it is also understood that aspects of the present invention are not limited to blood-glucose measurement systems and are applicable to broader diagnostic systems. Analytes that may be analyzed include glucose, lipid profiles (e.g., cholesterol, triglycerides, LDL and HDL), microalbumin, hemoglobin A1_{C} fructose, lactate, or bilirubin. It is contemplated that other analyte information may be determined (e.g., analyte concentrations). The analytes may be in, for example, a whole blood sample, a blood serum sample, a blood plasma sample, other body fluids like ISF (interstitial fluid) and urine, and non-body fluids.

While the invention is susceptible to various modifications and alternative forms, specific embodiments and methods thereof have been shown by way of example in the drawings and are described in detail herein. It should be understood, however, that it is not intended to limit the invention to the particular forms or methods disclosed, but, to the contrary, the intention is to cover all modifications, equivalents and alternatives falling within scope of the invention.

## Claims

1. A blood-glucose meter (110) for determining a blood-glucose concentration in a fluid sample, the blood-glucose meter configured to receive a test sensor (130), the blood-glucose meter (110) comprising:
a measurement system (112, 113) that determines a measurement of a concentration of a blood-glucose in a fluid sample received on the test sensor during a testing procedure;
a rechargeable battery (114); and
a connection (140) configured to electrically and mechanically couple the blood-glucose meter (110) to an external computing device including a power source (150);
**characterized in that** the rechargeable battery (114) is rechargeable with power from the power source (150), wherein the blood-glucose meter (110) receives the power via the connection (140), the connection (140) configured to electrically isolate the blood-glucose meter (110) from the power source (150) when the testing procedure is initiated while the blood-glucose meter (110) remains mechanically coupled to the computing device (120) via the connection (140).

2. The blood-glucose meter (110) of claim 1, wherein the testing procedure is initiated upon the blood-glucose meter (110) receiving the test sensor (130).

3. The blood-glucose meter (110) of claim 1, wherein the connection (140) includes an isolated DC-DC converter.

4. The blood-glucose meter (110) of claim 1, further comprising a microcontroller, the connection (140) including a transistor and a diode, the microcontroller configured to turn the transistor on and current flowing through the diode from the computing device when the test sensor is not received by the blood-glucose meter (110), and the microcontroller turning the transistor off and the blood-glucose meter (110) operating solely on power from the battery when the test sensor (130) is received by the blood-glucose meter (110).

5. The blood-glucose meter (110) of claim 5, wherein the transistor and the diode are configured to prevent the flow of current from the computing device when a high voltage occurs over the connection (140) due to a failure on the computing device.

6. The blood-glucose meter (110) of claim 1, wherein the connection (140) includes a silicon controlled rectifier (SCR), the SCR configured to turn off when a failure occurs on the computing device and a reversal of polarity occurs at the SCR with a resulting flow of alternating current.

7. The blood-glucose meter (110) of claim 1, wherein the connection (140) includes signal line connections and DC power line connections configured to transmit low voltage current from the computing device to the blood-glucose meter (110).

8. The blood-glucose meter (110) of claim 1, wherein the connection (140) includes first and second transistors with first and second diodes forwardly biased such that current flows through the diodes when the transistors are turned on.

9. The blood-glucose meter (110) of claim 8, wherein the blood-glucose meter (110) includes a microcontroller connected to the first and second transistors, and wherein the transistors are turned on when the microcontroller supplies minimum voltages to the base inputs of the transistors.

10. The blood-glucose meter (110) of claim 8, wherein the connection further includes first and second resistors connected to the first and second transistors, respectively.

## Patentansprüche

1. Blutzuckermessgerät (110) zum Bestimmen einer Blutzuckerkonzentration in einer Fluidprobe, wobei das Blutzuckermessgerät zum Aufnehmen eines Testsensors (130) eingerichtet ist, wobei das Blutzuckermessgerät (110) umfasst:
ein Messsystem (112, 113), das eine Messung einer Konzentration einer Blut-Glukose in einer Fluidprobe bestimmt, die auf dem Testsensor während eines Testvorgangs empfangen wird;
eine wiederaufladbare Batterie (114); und
eine Verbindung (140) eingerichtet zum elektrischen und mechanischen Koppeln des Blutzuckermessgeräts (110) mit einer externen Rechenvorrichtung, die eine Energiequelle (150) enthält
**dadurch gekennzeichnet, dass** die wiederaufladbare Batterie (114) mit Energie von der Energiequelle (150) wiederaufladbar ist, wobei das Blutzuckermessgerät (110) die Energie über die Verbindung (140) erhält, wobei die Verbindung (140) eingerichtet ist zum elektrischen Isolieren des Blutzuckermessgeräts (110) von der Energiequelle (150), wenn der Testvorgang eingeleitet wird, während das Blutzuckermessgerät (110) über die Verbindung (140) mechanisch mit der Rechenvorrichtung (120) gekoppelt bleibt.

2. Blutzuckermessgerät (110) nach Anspruch 1, wobei der Testvorgang eingeleitet wird, wenn das Blutzuckermessgerät (110) den Testsensor (130) empfängt.

3. Blutzuckermessgerät (110) nach Anspruch 1, wobei die Verbindung (140) einen isolierten DC-DC Wandler umfasst.

4. Blutzuckermessgerät (110) nach Anspruch 1, ferner umfassend einen Mikrocontroller, wobei die Verbindung (140) einen Transistor und eine Diode umfasst, wobei der Mikrocontroller eingerichtet ist zum Einschalten des Transistors und Strom durch die Diode von der Rechenvorrichtung fließt, wenn der Testsensor nicht von dem Blutzuckermessgerät (110) empfangen wird, und wobei der Mikrocontroller den Transistor ausschaltet und das Blutzuckermessgerät (110) ausschließlich mit Strom von der Batterie arbeitet, wenn der Testsensor (130) von dem Blutzuckermessgerät (110) empfangen wird.

5. Blutzuckermessgerät (110) nach Anspruch 5, wobei der Transistor und die Diode eingerichtet sind zum Verhindern des Stromflusses von der Rechenvorrichtung, wenn aufgrund eines Fehlers an der Rechenvorrichtung eine Hochspannung über der Verbindung (140) auftritt.

6. Blutzuckermessgerät (110) nach Anspruch 1, wobei der Anschluss (140) einen siliziumgesteuerten Gleichrichter (SCR) enthält, wobei der SCR eingerichtet ist zum Ausschalten, wenn ein Fehler an der Rechenvorrichtung auftritt und eine Umkehrung der Polarität am SCR mit einem resultierenden Wechselstromfluss auftritt.

7. Blutzuckermessgerät (110) nach Anspruch 1, wobei die Verbindung (140) Signalleitungsverbindungen und Gleichstromleitungsverbindungen umfasst, die eingerichtet sind zum Übertragen von einem Niederspannungsstrom von der Rechenvorrichtung zum Blutzuckermessgerät (110).

8. Blutzuckermessgerät (110) nach Anspruch 1, wobei die Verbindung (140) erste und zweite Transistoren mit ersten und zweiten Dioden umfasst, die in Durchlassrichtung vorgespannt sind, so dass Strom durch die Dioden fließt, wenn die Transistoren eingeschaltet sind.

9. Blutzuckermessgerät (110) nach Anspruch 8, wobei das Blutzuckermessgerät (110) einen Mikrocontroller umfasst, der mit den ersten und zweiten Transistoren verbunden ist, und wobei die Transistoren eingeschaltet werden, wenn der Mikrocontroller Mindestspannungen an die Basiseingänge der Transistoren liefert.

10. Blutzuckermessgerät (110) nach Anspruch 8, wobei die Verbindung ferner einen ersten und einen zweiten Widerstand umfasst, die mit dem ersten bzw. dem zweiten Transistor verbunden sind.

## Revendications

1. Dispositif de mesure de glucose sanguin (110) pour déterminer une concentration de glucose sanguin dans un échantillon de fluide, l'dispositif de mesure du glucose sanguin étant configuré pour recevoir un capteur de test (130), le dispositif de mesure de glucose sanguin (110) comprenant :
- un système de mesure (112, 113) qui détermine une mesure d'une concentration d'un glucose sanguin dans un échantillon de fluide reçu sur le capteur de test pendant une procédure de test ;
- une batterie rechargeable (114) ; et
- une connexion (140) configurée pour coupler électriquement et mécaniquement le dispositif de mesure de glucose sanguin (110) à un dispositif informatique externe comprenant une source d'énergie (150) ;
**caractérisé**
**en ce que** la batterie rechargeable (114) est rechargeable avec de l'énergie provenant de la source d'énergie (150), où le dispositif de mesure de glucose sanguin (110) reçoit l'énergie par l'intermédiaire de la connexion (140), la connexion (140) étant configurée pour isoler électriquement le dispositif de mesure de glucose sanguin (110) de la source d'énergie (150) lorsque la procédure de test est lancée tandis que le dispositif de mesure de glucose sanguin (110) reste mécaniquement couplé au dispositif informatique (120) par l'intermédiaire de la connexion (140).

2. Dispositif de mesure de glucose sanguin (110) selon la revendication 1, où la procédure de test est lancée lorsque le dispositif de mesure de glucose sanguin (110) reçoit le capteur de test (130).

3. Dispositif de mesure de glucose sanguin (110) selon la revendication 1, où la connexion (140) comprend un convertisseur DC-CC isolé.

4. Dispositif de mesure de glucose sanguin (110) selon la revendication 1, comprenant en outre un microcontrôleur, la connexion (140) comprenant un transistor et une diode, le microcontrôleur étant configuré pour activer le transistor et le courant circulant à travers la diode depuis le dispositif informatique lorsque le capteur de test n'est pas reçu par le dispositif de mesure de glucose sanguin (110), et le microcontrôleur désactivant le transistor et le dispositif de mesure de glucose sanguin (110) fonctionnant uniquement sur l'alimentation de la batterie lorsque le capteur de test (130) est reçu par le dispositif de mesure de glucose sanguin (110).

5. Dispositif de mesure de glucose sanguin (110) selon la revendication 5, où le transistor et la diode sont configurés pour empêcher la circulation du courant depuis le dispositif informatique lorsqu'une haute tension se produit sur la connexion (140) en raison d'une défaillance du dispositif informatique.

6. Dispositif de mesure de glucose sanguin (110) selon la revendication 1, où la connexion (140) comprend un redresseur commandé au silicium (SCR), le SCR étant configuré pour s'éteindre lorsqu'une défaillance se produit sur le dispositif informatique et qu'une inversion de polarité se produit au niveau du SCR avec un flux de courant alternatif résultant.

7. Dispositif de mesure de glucose sanguin (110) selon la revendication 1, où la connexion (140) comprend des connexions de lignes de signaux et des connexions de lignes d'alimentation en courant continu configurées pour transmettre un courant basse tension du dispositif informatique au dispositif de mesure de glucose sanguin (110).

8. Dispositif de mesure de glucose sanguin (110) selon la revendication 1, où la connexion (140) comprend des premier et second transistors avec des première et seconde diodes polarisées dans le sens direct, de sorte que le courant circule à travers les diodes lorsque les transistors sont allumés.

9. Dispositif de mesure de glucose sanguin (110) selon la revendication 8, où le dispositif de mesure de glucose sanguin (110) comprend un microcontrôleur connecté aux premier et second transistors, et où les transistors sont activés lorsque le microcontrôleur fournit des tensions minimales aux entrées de base des transistors.

10. Dispositif de mesure de glucose sanguin (110) selon la revendication 8, où la connexion comprend en outre des première et deuxième résistances connectées aux premier et deuxième transistors, respectivement.
